Europäisches Patentamt

(19) European Patent Office

Office européen des brevets

(11) **EP 0 721 799 B1**

(12) **EUROPEAN PATENT SPECIFICATION**

(45) Date of publication and mention
of the grant of the patent:
**08.12.1999 Bulletin 1999/49**

(51) Int. Cl.$^6$: **B01J 23/80**, C01B 3/16,
C07C 29/154

(21) Application number: **95110290.4**

(22) Date of filing: **30.06.1995**

(54) **Promoted and stabilized copper oxide and zinc oxide catalyst and preparation method**

Aktivierter und stabilisierter Kupferoxid- und Zinkoxidkatalysator und Verfahren zu seiner Herstellung

Catalyseur en oxyde de cuivre et oxyde de zinc stabilisé et activé et procédé pour sa préparation

(84) Designated Contracting States:
**DE DK FR GB NL**

(30) Priority: **11.01.1995 US 371500**

(43) Date of publication of application:
**17.07.1996 Bulletin 1996/29**

(73) Proprietor:
**United Catalysts, Inc.**
**Louisville Kentucky 40232 (US)**

(72) Inventors:
• **Wagner, Jon P.**
**Louisville, Kentucky 40222 (US)**
• **Hu, X.D.**
**Louisville, Kentucky 40241 (US)**

(74) Representative:
**Patentanwälte**
**Dipl.-Ing. R. Splanemann**
**Dr. B. Reitzner**
**Dipl.-Ing. K. Baronetzky**
**Tal 13**
**80331 München (DE)**

(56) References cited:
**EP-A- 0 155 868**          **EP-A- 0 296 734**
**EP-A- 0 482 753**

• **JOURNAL OF FUEL CHEMISTRY AND
TECHNOLOGY, vol. 20, no. 2, June 1992, pages
146-152, XP002000915 LI CHENGYUE ET AL.:
"reaction network of higher alcohol synthesis
on a cu-zn-al-ti catalyst promoted with k2co3"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

Background of Invention

1. Field of Invention.

This invention is directed to catalysts useful in the conversion of carbon oxides. Specifically it is directed to catalyst useful in the conversion of carbon oxides and water for the production of hydrogen.

2. Prior Art.

Synthesis gas represents one of the most important feedstocks of the chemical industry. It is used to synthesize basic chemicals, such as methanol or oxyaldehydes, as well as for the production of ammonia and pure hydrogen. However, synthesis gas produced by steam reforming of hydrocarbons does not meet the requirements for further use in some cases because the $CO/H_2$ ratio is too high. It is therefore industrial practice to reduce the CO content by conversion with steam.

Hydrogen is an indispensable component for many petroleum and chemical processes. Refineries in the petroleum industry, and methanol and ammonia plants in the chemical industry consume considerable quantities of hydrogen during processes for the production of gasoline and fertilizers. As environmental regulations demand cleaner, renewable and non-polluting processes and products, most of the hydrogen balances at petroleum refineries are becoming negative. As laws mandate lower aromatics in gasoline and diesel fuels, $H_2$ is now consumed in aromatic saturation and thus, less $H_2$ is available as a by-product. At the same time, $H_2$ consumption is increasing in hydro-treating units in the refineries because many of these same laws require low sulfur level in fuels.

Hydrogen is primarily obtained by steam reforming methane or mixture of hydrocarbons, a reaction which produces hydrogen, carbon dioxide and carbon monoxide. To improve $H_2$ yield and also the operating efficiency of carbon monoxide conversion, the water gas shift reaction is extensively used in commercial hydrogen or ammonia plants. The reaction can be described as:

$$CO + H_2O \rightleftharpoons CO_2 + H_2 \quad H=-9.84 \text{ Kcal/mol at } 298° \text{ K}$$

For maximum $H_2$ yield and CO conversion efficiency, the water gas shift reaction is usually carried out in two stages: at high temperatures typically 350-400°C and at low temperature typically 180-240°C.

While lower temperatures favor more complete carbon monoxide conversion, higher temperatures allow recovery of the heat of reaction at a sufficient temperature level to generate high pressure steam. For maximum efficiency and economy of operation, many plants contain a high temperature reaction unit for bulk carbon monoxide conversion and heat recovery, and a low temperature reaction unit for final carbon monoxide conversion.

Chromium-promoted iron catalysts are normally used in the first stage at temperatures above about 350°C to reduce the CO content to about 3-4% (see, for example, D.S. Newsom, Catal. Rev., 21, p. 275 (1980)). As is known from the literature (see for example, H. Topsoe and M. Boudart, J. Catal., 31, p. 346 (1973)), the chromium oxide promoter combines two functions. In the first place, it serves to enhance catalytic activity and in the second place, it acts as a heat stabilizer, i.e., it increases the heat stability of magnetite, the active form of the catalyst, and prevents unduly rapid deactivation under conditions of technical use.

Unfortunately, when chromium is used, especially in hexavalent form, expenditures must be incurred to guarantee worker safety both during production and later handling of the catalyst, and health hazards cannot be fully ruled out despite considerable effort. In addition, the spent catalyst ultimately poses a hazard to man and the environment and must be disposed of with allowance for the provisions in force for toxic waste.

The commonly used catalysts for water gas shift reaction at low temperature (or so-called low temperature shift reaction) in industry contain copper oxide, zinc oxide and aluminum oxide. Because these catalysts operate at relatively low temperature, they generate equilibrium carbon monoxide concentrations of less than 0.3% in the exit gas stream over an active low temperature shift catalyst. However, performance of carbon monoxide conversion and hydrogen yield gradually decreases during normal operations as a result of deactivation of the catalyst. This deactivation is caused by poisoning, generally from traces of chloride and sulfur compounds in the feed and the hydrothermal environment of the reaction. The rate of the hydrothermal deactivation, in particular, is dependent on reaction conditions such as temperature, steam to gas ratio and composition of the feed gas mixture, and is closely dependent on the formulation and manufacturing process of the catalyst.

A typical composition of a low temperature shift catalyst is comprised of from 30 to 60% of CuO, 20 to 50% of ZnO and 5-40% of $Al_2O_3$. The catalyst is usually made by either co-precipitation of metal salts (nitrate, sulfate, or acetate), thermal decomposition of metal complexes, or impregnation of metal salt onto a carrier. Depending on the preparation conditions (pH, temperature, addition rate and composition), one or several of the following mixed copper/zinc hydroxy carbonate phases are present in the precursor of the catalyst: (a) malachite $Cu_2CO_3(OH)_2$, (b) rosasite $(Cu,Zn)_2CO_3(OH)_2$, (c) hydrozincite $Zn_5(CO_3)_2(OH)_6$, (d) aurichalcite $(Cu,Zn)_5(CO_3)_2(OH)_6$, and (e) hydrotalcite $(Cu,Zn)_6Al_2(OH)_{16}CO_3$. The catalyst is then washed to remove foreign ions, dried and calcined at an appropriate temperature to form oxides. With appropriate precursors and preparation conditions, a mixed copper/zinc oxide phase rather than segregated cupric oxide and zinc oxide will be formed during calcination at 250-450°C. The catalyst must be reduced with hydrogen at 100-300°C before being put on stream. During reduction, copper oxide in cupric form is reduced to either metallic copper or/and cuprous oxide.

It is well accepted that reduced copper is an active species for low temperature shift catalyst. The reaction is initiated by adsorption of water and carbon monoxide molecules, proceeds with dissociation of water, and completes with association of the adsorbed intermediates to form hydrogen and carbon dioxide. All the steps mentioned above are carried out on the surface of copper active sites. In general, copper-based catalysts are very susceptible to thermal sintering which results in a loss of copper surface area and therefore activity. This situation arises because of high dispersion of the reduced copper on the catalyst and high mobility of the highly dispersed copper crystallites. With the presence of steam under the reaction environment, a rapid loss of copper surface area occurs as a result of sintering. Under some extreme catalyst testing conditions (high steam to carbon ratio), 30 to 50% of the original copper surface area may be lost in a test of 10 to 15 days depending upon the formulation and preparation method. It is believed that an industrial low temperature shift catalyst copper is partially stabilized by zinc oxide and aluminum oxide. In the presence of significant partial pressures of steam as in the low temperature shift conditions, zinc oxide selectively adsorbs water. Migration and/or inclusion of copper into a zinc oxide matrix inhibits copper crystallite growth. In addition, zinc oxide protects copper from poisoning of chloride and sulfur. The appropriate incorporation of aluminum in the matrix of copper oxide and zinc oxide can further increase the hydrothermal stability of copper.

As mentioned above, standard catalysts for this conversion stage are based on Cu-Zn oxide. See, for example, U.S. Patent No. 1,809,978. This type of catalyst has a major drawback of extremely low heat stability so that its use is essentially limited to temperatures below about 250°C. Further catalyst developments have focused on conversion activity at lower temperatures. See U.S. Patent No. 3,303,001.

U.S. Patent No. 4,308,176 describes catalysts for conversion of carbon monoxide based on copper oxide and/or zinc oxide on aluminum oxide spinals, wherein the catalyst is improved by the incorporation of zinc oxide into the pores of the spinel structure. U.S. Patent No. 3,922,337 discloses a low-temperature carbon monoxide shift catalyst containing copper and zinc oxides, wherein a sodium alkalized alumina improves resistance against halogen poisoning. See also U.S. Patent No. 3,518,208.

European Patent No. 0 296 734 B1 discloses a copper containing catalyst for carbon monoxide conversion. The catalyst is formed from copper oxide and one or more other oxidic materials generally including zinc oxide. The catalyst may also contain oxides of at least one other element selected from the group of aluminum, vanadium, chromium, titanium, zirconium, thorium, uranium, molybdenum, tungsten, manganese, boron, and the rare earth elements. Preferably, alumina is used. The catalyst produced possesses high specific copper surface area outside the range of conventional, unpromoted copper/zinc catalyst. See also U.S. Patent No. 4,711,773.

Copper-containing catalysts for low temperature shift conversion may also include a potassium component to suppress the formation of by-products, such as amines and methanol as disclosed in U.S. Patent No. 5,128,307. A similar type of catalyst may also be "alkali doped" as disclosed in U.S. Patent No. 5,021,233.

In addition, catalysts for the reaction of carbon monoxide with steam containing copper, zinc, and at least one metal selected from manganese and the metals of Groups II to V on the Periodic Table wherein those metals preferably include aluminum or magnesium, although titanium or zirconium or thorium can be used are disclosed in G.B. 1,131,631.

Catalysts for use in carbon monoxide shift reactions comprised of a copper/zinc/alumina precursor and another metal selected from the group of lanthanum, cerium, or zirconium are disclosed in U.S. Patent No. 4,835,132.

U.S. Patent No. 4,683,218 discloses a catalyst for a water gas shift reaction comprised of zinc, copper, an element from the lanthanum group and from the rare earth group.

EP-A-0 155 868 (US-A-4 552 861) discloses a process for the manufacture of a catalyst containing copper, zinc, aluminium and at least one metal from the group of (a) rare earth elements and (b) zirconium.

EP-A-0 482 753 discloses a method of forming a methanol synthesis catalyst precursor which method comprises forming a precipitate comprising compounds, thermally decomposable to oxides or mixed oxides, of copper, zinc, aluminium and at least one element of Group IVB and/or Group VIIB of the Periodic Table of Elements. Zirconium is the preferred Group IVB element. The amount of titanium in the catalyst is not disclosed.

Li Chengyue et al. in the "Journal of Fuel Chemistry and Technology", vol. 20, no. 2, June 1992, pages 146-152, disclose a "Reaction Network of Higher Alcohol Synthesis on a Cu-Zn-Al-Ti Catalyst Promoted with $K_2CO_3$", wherein the percentages listed for the quantity of any of the compounds are not disclosed. Furthermore, since no calcination temperatures are reported, it is not clear whether the components are present in the oxide form.

Although copper is physically and physicochemically stabilized by both zinc oxide and aluminum oxide and attempts of further stabilization of the catalyst have been made as taught by prior art, sintering of copper crystallite is still a main cause for deactivation/aging of the catalyst, especially when there are very low concentration of poison in the feed. For example, the copper crystallite size of a fresh catalyst ranges from 30-100 angstroms in contrast with 100-1,000 angstroms over a discharged used catalyst from the plant. The known catalysts thus need to be improved with regard to activity and stability.

A major reason for lack of superior activity and hydrothermal stability over the known catalyst is due to lack of significant electronic modifications and interactions among copper, zinc oxide and aluminum oxide. One or several highly thermally stable component(s) is(are) thus needed to act as a promoter/stabilizer of the catalyst and it(they) should have one or several of the following functions:

(1) Modify the catalyst into a suitable morphological form with typical particle size from 20-200 μm;

(2) Modify the catalyst with desirable pore structure with peak of the pore distribution at 5 to 20 nm (50-200 Å) and the total pore volume at 0.2-0.4 $cm^3$/g;

(3) Modify the catalyst with desirable BET surface area in the neighborhood of 40 to 200 $m^2$/g;

(4) Construct and reinforce the framework of the catalyst so that the catalyst can maintain its physical integrity and strength under certain mechanical, thermal, and/or reaction forces encountered in industrial applications; and

(5) Separate the Cu/ZnO crystallites and put appropriate space among them so that the Cu/ZnO pair is able to be well dispersed throughout the catalyst structure.

(6) Allow electron transfer between copper and the promoter and enhance interaction among the components.

The objective of the present invention thus is a catalyst for a CO conversion process that has superior activity and hydrothermal stability.

It is a further object of the present invention to produce a long life low temperature water gas shift catalyst.

It is a still further object of the present invention to prepare a catalyst for a CO conversion process that exhibits significant hydrogen production over the lifetime of the catalyst.

It is a still further object of this invention to produce a catalyst that can be used for other processes in which carbon oxides are converted to methane and/or methanol.

(c) Summary of Invention

In accordance with the present invention there is provided a catalyst useful in the conversion of carbon monoxide with the chemical composition mentioned below.

The invention is also a process for low temperature shift conversion of carbon monoxide by use of the above referenced catalyst.

The catalyst of this invention is preferably used in a process in which carbon monoxide and water are converted to carbon dioxide and hydrogen at temperatures in the range between 150° and 350° C, under pressures from 1.5 to 70 bars at dry gas space velocities from 5,000 to 30,000 $hr^{-1}$ and at steam to gas ratio from 0.3 to 2.

(d) Description of the Invention

The carbon oxide conversion catalyst, before reductive activation, measured by weight on a dry weight basis after calcination at a temperature of at least 600°C, comprises 30 - 70 % by weight CuO, preferably 30 - 60 % by weight CuO, 20 - 50 % by weight ZnO, preferably 20 -50 % by weight ZnO, 5 - 40 % by weight of aluminum oxide, preferably 5 - 20 % by weight of amuminum oxide, 0.1 - 20 % by weight, preferably 0.2 - 10 % by weight of an oxide of titanium (as a promotor/stabilizer), and 50 - 100 ppm of a Group I-A element in an oxide form, preferably potassium oxide and/or cesium oxide (as a copromoter/stabilizer). While the preparation of the copper and zinc oxide components is conventional, the nature and amount of the promoters/stabilizers utilized in the catalyst and the catalyst's method of production are important to the operation of the catalyst.

The amount of promoter/stabilizer utilized in the catalyst needs to be controlled within certain limitations. In the case of titanimum, this level ranges from 0.1 to 20%, preferably 0.2-10% by weight, depending on the method of preparation. For example, if the promoter is added to the mixture of the metal salts in coprecipitation, the amount of the promoter should be at the high end of the range, whereas if the promoter is added to the surface of a finished catalyst using the impregnation method, it should be at the low end of the range. Below this range, insufficient amounts of the promotor are available to react, whereas above this range, the effective copper surface is reduced by the promoter and an insufficient amount of active sites is available for significant occurrence of the reaction. If an excess amount of the titanium oxide promoter is utilized, the titanium can behave as a suppressant to the activity of the catalyst.

The chemical components containing Group I-A elements can be chosen from hydroxide, bicarbonate, carbonate, chloride, bromide, acetate, citrate, oxalate, sulfate and nitrate, preferably, hydroxide, oxalate, bicarbonate, carbonate or nitrate. The titanium promoter can be prepared from oxide, hydroxide, chloride, sulfate, isopropoxide and protoxide, preferably, oxide, hydroxide and sulfate. As C, Cl and S are contaminants for the catalyst, a thorough washing and/or ion-exchange is important in preparation of the catalyst.

A homogeneous mixture of the promoter and the copromoter with CuO/ZnO ensures an intimate interaction among the components. It is advantageous to prepare the catalyst using one or combination of the following methods: coprecipitation, decomposition, impregnation and mechanical mixing, preferably coprecipitation and decomposition. The method chosen should guarantee intense blending of the components. In coprecipitation, the catalyst is prepared by mixing the acidic metal salt of copper, zinc, aluminum, the appropriate Group I-A compound and the appropriate titanium compound with a basic carbonate or bicarbonate solution at a pH range from 6-9 and a temperature range from ambient to 80°C, washing the precipitate at a temperature ranging from ambient to 50°C, filtering at ambient temperature, drying at a temperature range from 100 to 160°C, calcinating at a temperature from 370 to 500°C and finally forming the product into desirable size and geometric shape.

Heat treatment or calcination can be conducted under static conditions, for example, in a tray furnace, or under dynamic conditions, such as in a rotary kiln. The temperatures and residence times are determined for each individual type of catalyst.

U.S. Patent No. 3,615,217 teaches the process of the decomposition method. In preparation of the decomposition solution, a copper and zinc complex solution is mixed with proper forms of aluminum compounds and compounds of Group I-A elements and of titanium which are generally less soluble in an aqueous solution. The solution is then heated up to 120°C in a container either under vacuum or ambient pressure, typically under slightly positive pressure, and the solid is obtained until all the ammonia and a significant portion of the carbon dioxide is released. Post-treatment of the solid similar to the procedure used in coprecipitation leads to formation of the final catalyst. Impregnation of the solution containing the Group I-A inorganic or organic element and/or titanium compounds, preferably organic compounds, onto the catalyst can be carried out at any stage of the preparation, it being advantageous to spray or mix the solution containing promoter/stabilizer and copromoter/stabilizer onto the solid slurry after aging, washing, filtration or drying. A further drying after impregnation is needed to ensure the completion of decomposition of the promoter compounds.

The BET surface of the catalyst is at least 40 to 200 $m^2$/g, and, preferably, 80 to 140 $m^2$/g. The BET surface is determined by $N_2$ adsorption according to the single-point method, as described in DIN 66 132.

The specific pore volume of the catalyst determined by Hg porosimetry is from 0.2 $cm^3$/g to 0.4 $cm^3$/g. The specific pore volume is determined according to the mercury penetration method described in J. Van Brakel, et al., Powder Technology, 29, p.1 (1981). In this method, mercury is pressed up to a pressure of about 4000 bar into the catalyst moldings, during which the volume reduction of the mercury is plotted as a function of pressure. A curve is obtained from which the pore distribution can also be determined. According to this mercury penetration method, only the volume and distribution of pores with a diameter of >3.6 nm can be determined.

Known methods can be used to form the catalyst mass. Preferred forming methods are pelletizing and extrusion, in which the use of inorganic or organic auxiliaries as lubricants or to improve plasticity during extrusion is recommended. Forming can also be undertaken both before and after calcination.

The catalysts preferably occur as moldings, especially in the form of spheres, pellets, rings, tablets or extruded products, in which the latter are formed mostly as solid or hollow objects in order to achieve high geometric surface with a simultaneously low resistance to flow. Honeycombs are particularly preferred shapes.

The catalyst is preferably employed in a process in which carbon monoxide and water are converted in the temperature range between 150° and 350° C, under pressures from 1.5 to 70 bars at dry gas space velocity of 5000 to 30,000 $hr^{-1}$ and at a steam to gas ratio of about 0.3 to 2.0 carbon.

Although the catalyst is preferably utilized in a process in which carbon monoxide and water are converted to carbon dioxide and hydrogen, other process utilizing copper-based catalysts can also be enhanced by use of the catalyst. For example, the catalyst can be used for converting carbon monoxide and/or carbon dioxide to methanol and/or higher alcohols in the presence of hydrogen and other inert gases at temperatures in a range of 200° to 400°C. Further, the catalyst can be used for the removal of traces of oxygen, hydrogen, chloride and/or carbon monoxide from a gas stream. In addition, the catalyst can be used for general hydrogenation processes in which conventional copper-based catalysts are used.

The catalyst according to this invention makes it possible to carry out the process under the above described conditions of low temperature shift in greater efficiency than the known catalysts, typically 5 to 20% higher in activity, 5-15% longer in life and therefore, 10-40% higher in total $H_2$ production over the lifetime of the catalyst.

The following examples describe production and use of the catalysts employed according to the invention, and of comparison catalysts

**Example 1 (Comparison)**

A copper solution was first prepared with 254 g of copper being added to a solution containing 728 g of 28% $NH_4OH$ and 316 g of $NH_4HCO_3$, and at least 4882 ml of water being needed for every 1000 g of copper. The copper solution is allowed to sit at 70 to 78°C with vigorous agitation and with fast $O_2/N_2$ gas flow, the $O_2$ concentration in the gas mixture being 10% or higher. A zinc solution was then prepared in a similar manner with a slightly different recipe using 523 g of zinc added to a solution of 1456 g of $NH_4OH$, 632 g of $NH_4HCO_3$ and 2620 g of water. 1973 g of the copper solution was mixed with 1414 g of the zinc solution under agitation. The combined copper zinc solution was rapidly reacted with 80 grams of alumina in a 14 liter reactor at 77-85°C in a fashion such that the temperature increase was gradual until the color of the solution turned from dark blue to light blue gray. During reaction, agitation must be kept vigorous. The solid was then filtered with a two-liter Buchner funnel and a four-liter filtration flask with vacuum being provided by a Venturi water suction device. The resulting solid was dried at 150°C overnight and calcined in controlled atmosphere at 370°C. The catalyst tablets of 4.76 x 2.38 mm were formed by pressing the solid powder together with 2% graphite. The physical and chemical properties of the resulting catalyst (EX1) is summarized in the Table 1.

**Example 2 (Comparison)**

The preparation of the copper and zinc solution followed the description in Example 1. The combined copper/zinc solution was added to a slurry containing 70 g of alumina and 25 g of zirconia at a rate of 40 ml/min. The reaction temperature was gradually increased from 70°C to 90°C with agitation. The post-treatment of the resulting slurry followed Example 1. The physical and chemical properties of the resulting catalyst (EX2) are listed in Table 1.

**Example 3**

The preparation of the catalyst (EX3) followed the descriptions in Examples 1 and 2 except a mixture of $Al_2O_3$-$TiO_2$ was prepared. The potassium solution was prepared by dissolving a predetermined amount of potassium oxalate powder containing 1.5 g of potassium in double de-ionized water. The amount of potassium oxalate used is determined by the grade and hydration level of the material. The potassium solution was then mixed with 1000 g of titania. The titania slurry was maintained under agitation for at least 10 minutes, and then filtered in the same apparatus described in Example 1 repeatedly after being re-slurred with additional double de-ionized water and dried at 120°C overnight. To prepare the catalyst, EX3, it took 15 g of the dried potassium modified titania and 80 g of alumina. BET surface area of the catalyst promoted with titania and potassium was 120 $m^2$/g. The remaining information can be found in Table 1.

**Example 4**

The preparation of the catalyst (EX4) followed the methods found in EX1, EX2 and EX3 except 37.5 g of potassium containing titania was used.

**Example 5**

The preparation of the catalyst EX5 followed the description in Example 3 except using titanium oxysulfate instead of titanium oxide. The amount of titanium oxysulfate used in this example was 61g

**Example 6**

The preparation of the catalyst EX6 followed the description in Example 3 except using titanium chloride. 95 g of titanium chloride was mixed with the copper, zinc, and aluminum solution.

**Example 7**

2.3 g of titanium isopropoxide was carefully added into 7.4 g of n-hexane under nitrogen atmosphere to make Solution A. The Solution A was then slowly added to EX1 powder and allowed to be evacuated under nitrogen for 60 minutes. The resulting material was dried with flowing air at a rate of 5 liter/minutes. The temperature of drying started at 26°C and was gradually brought to 71°C in a three hour period. The material was then calcined at 370°C for two hours and made into catalyst E7.

Table 1

| Physical and Chemical Properties of EX1-EX7 Catalysts | | | | | | | |
|---|---|---|---|---|---|---|---|
| Catalyst XRD | EX1 | EX2 | EX3 | EX4 | EX5 | EX6 | EX7 |
| CuO MCS | 71 | 32 | 55 | 68 | 205 | <10 | 56 |
| ZnO MCS | <10 | 67 | 58 | 39 | <10 | <10 | 55 |
| Bulk Density g/cm$^3$ (lb/cubic feet) | 1.17 (72.9) | 1.26 (79.0) | 1.31 (81.5) | 1.40 (87.6) | 1.25 (78.1) | 1.26 (78.5) | 1.26 (78.4) |
| Surface Area (mm$^2$/g) | 96 | 108 | 120 | 97 | 94 | 85 | 105 |
| Pore Volume (cm$^3$/g) | 0.36 | 0.31 | 0.26 | (not available) | | | |
| Crush g Strength (lb) | 6810 (15) | 8172 (18) | 10896 (24) | 21338 (47) | 11350 (25) | 11350 (25) | 12712 (28) |
| (DWL Dead Weight Load) | | | | | | | |

**Test Data**

CO conversion activity was measured by passing a gas described in Table 2 over the catalyst at a pressure of 10.3 bar and a temperature of 204°C. The dry gas space velocity was 11,250 volumes of gas per hour per volume of catalyst (hr$^{-1}$), and the steam to gas ratio was 1.5 on a molar basis. Before the activity analysis the catalysts were loaded into a laboratory test reactor and pretreated to obtain the active species. The pretreatment consisted of heating the catalyst in a flowing stream of hydrogen and nitrogen at a pressure of 3.5 bar to a final temperature of 204°C which was maintained for 16 hours.

Table 2

feed gas composition

3.0% CO

17.0% $CO_2$

2.0% $N_2$

68.0% $H_2$

The catalysts: EX1 made according to Example 1, and EX3 made according to Example 3 were evaluated for activity in terms of CO conversion as defined below:

%CO conversion = {(moles CO inlet - moles CO exit) / moles CO inlet} * 100%

The activity in terms of CO conversion was measured after the catalyst were exposed to the feed gas for 4 hours to establish steady state conversion. The exit gas was analyzed by gas chromatography after the residual water had been condensed from the product gases. The results are summarized in Table 3.

Table 3

| | EX1 | EX3 |
|---|---|---|
| %CO Conversion | 89.2 | 93.1 |

Higher CO conversion increases ammonia and methanol plant's efficiencies because more $H_2$ is produced and

therefore there is less CO to methanate which in turn will consume less $H_2$. Higher CO conversion will also decrease the purge rate in the synthesis loop resulting in less $H_2$ loss in the purge gas. In ammonia and methanol plants the production rate increases with increased hydrogen production.

Due to the nature of the carbon monoxide conversion reaction, equilibrium is favored at lower temperatures. A catalyst that exhibits high CO conversion activity can be operated at lower temperatures where the reaction equilibrium can be exploited. Operating at lower temperatures would also decrease the rate of deactivation caused by thermal affects, i.e. decreased copper sintering rate.

To measure the CO conversion stability, the feed gas as described in Table 2 was maintained for long periods of time while the exit gas was monitored routinely. Data from the extended tests are summarized in Table 4.

Table 4

| %CO Conversion Stability | | | |
|---|---|---|---|
| Hours on Stream | EX1 | EX3 | EX4 |
| 4 | 88.9 | 91.3 | 90.3 |
| 28 | 87.8 | 90.1 | 89.8 |
| 124 | 85.1 | 88.5 | 88.4 |
| 244 | 82.0 | 87.6 | 86.3 |
| 364 | 79.9 | --- | 85.1 |

As the data indicate, the catalyst(s) prepared by the method described in Example 1 (Comparison) did not maintain the high CO conversion with time on stream. In comparison the catalysts prepared by the method described in Examples 3 and 4 exhibited better activity stability as time on stream increases. In industrial applications, this is an accumulative effect which will correspond to longer lives with higher hydrogen production throughout the entire life of the catalyst.

The CO conversion stability can be confirmed with the analysis of the catalysts after the extended stability test. As discussed previously, one of the major causes of deactivation of the copper catalysts is the sintering of the copper crystallites. X-ray diffraction is an industry accepted method for determining the average size of crystalline compounds that exceed 3.0 nm. Data collected on the spent catalysts is summarized in Table 5 and expressed in terms of mean crystallite size in nanometers (mcs, nm).

Table 5

| X-Ray Diffraction Data | | |
|---|---|---|
| | Hours on Stream | After Test Cu° (mcs, nm) |
| EX1 | 244 | 25.1 |
| EX3 | 244 | 16.0 |
| EX1 | 364 | 27.6 |
| EX4 | 364 | 15.0 |

The data shows that the catalysts prepared by the methods described here exhibit significantly lower copper crystallite growth compared to catalyst EX1.

**Claims**

1.  A carbon oxide conversion catalyst which, before reductive activation measured by weight on a dry weight basis after calcination at a temperature of at least 370°C, comprises 30-70% by weight CuO, 20-50% by weight ZnO, 5-40% by weight of aluminum oxide, 0.1-20% by weight of an oxide of titanium. and 50-1000 ppm of a Group I-A element in an oxide form.

2.  The catalyst of Claim 1 which comprises 30-60% by weight CuO, 20-50% by weight ZnO, and 5 to 20% by weight of aluminum oxide.

3. The catalyst of Claims 1 or 2 wherein the Group I-A element is selected from the group consisting of potassium and cesium.

4. The catalyst of any one of Claims 1 to 3 containing 0.2 to 10% by weight of an oxide of titanium.

5. The catalyst of any one of Claims 1 to 4 wherein prior to calcination the titanium is in a form selected from the group consisting of an oxide, hydroxide, chloride, sulphate, isopropoxide and propoxide.

6. The catalyst of any one of Claims 1 to 5 wherein prior to calcination the Group I-A element is in a form selected from the group consisting of hydroxide, bicarbonate, carbonate, chloride, bromide, acetate, citrate, oxalate, sulphate and nitrate.

7. The catalyst of any one of Claims 1 to 6 in the form of spheres, pellets, tablets, rings, solid, hollow or shaped extrudates.

8. A method for preparation of a carbon monoxide conversion catalyst which, before reductive activation measured by weight on a dry weight basis after calcination at a temperature of at least 370°C, comprises 30 to 70 weight percent CuO, 20 to 50 weight percent ZnO, 5 to 40 weight percent of $Al_2O_3$, 0.1 to 20 weight percent of titanium and 50 to 1000 ppm of a Group I-A element in an oxide form, wherein the method is selected from a group consisting of coprecipitation, decomposition, impregnation and mechanical mixing.

9. A process for converting carbon monoxide or carbon dioxide to an alcohol in the presence of hydrogen and inert gases in a gas stream at a temperature of 200° to 400°C using the catalyst of any one of Claims 1 to 7.

10. A process for the removal of traces of oxygen, hydrogen, chloride and carbon monoxide from a gas stream using the catalyst of any one of Claims 1 to 7.

11. A hydrogenation process using the catalyst of any one of Claims 1 to 7.

12. The process of converting carbon oxide to carbon dioxide with water using the catalyst of any one of claims 1 to 7.

**Patentansprüche**

1. Katalysator zur Konvertierung von Kohlenmonoxid, der vor der reduktiven Aktivierung, gemessen nach dem Gewicht auf der Basis des Trockengewichts nach der Calcinierung bei einer Temperatur von mindestens 370°C, 30 bis 70 Gew.-% CuO, 20 bis 50 Gew.-% ZnO, 5 bis 40 Gew.-% Aluminiumoxid, 0,1 bis 20 Gew.-% eines Oxids des Titans und 50 bis 1.000 ppm eines Elements der Gruppe I-A in einer oxidischen Form enthält.

2. Katalysator nach Anspruch 1, der 30 bis 60 Gew.-% CuO, 20 bis 50 Gew.-% ZnO und 5 bis 20 Gew.-% Aluminiumoxid enthält.

3. Katalysator nach Anspruch 1 oder 2, worin das Element der Gruppe I-A aus der Gruppe, bestehend aus Kalium und Cäsium, ausgewählt ist.

4. Katalysator nach einem der Ansprüche 1 bis 3, enthaltend 0,2 bis 10 Gew.-% eines Oxids des Titans.

5. Katalysator nach einem der Ansprüche 1 bis 4, worin das Titan vor der Calcinierung in einer Form vorliegt, die aus der Gruppe, bestehend aus einem Oxid, Hydroxid, Chlorid, Sulfat, Isopropoxid und Propoxid, ausgewählt ist.

6. Katalysator nach einer der Ansprüche 1 bis 5, worin das Element der Gruppe I-A vor der Calcinierung in einer Form vorliegt, die aus der Gruppe, bestehend aus Hydroxid, Bicarbonat, Carbonat, Chlorid, Bromid, Acetat, Citrat, Oxalat, Sulfat und Nitrat, ausgewählt ist.

7. Katalysator nach einem der Ansprüche 1 bis 6 in Form von Kugeln, Pellets, Tabletten, Ringen, Festkörpern, hohlen oder geformten Extrudaten.

8. Verfahren zur Herstellung eines Katalysators zur Konvertierung von Kohlenmonoxid, der vor der reduktiven Aktivierung, gemessen nach dem Gewicht auf der Basis des Trockengewichts nach der Calcinierung bei einer Temperatur von mindestens 370°C, 30 bis 70 Gew.-% CuO, 20 bis 50 Gew.-% ZnO, 5 bis 40 Gew.-% $Al_2O_3$, 0,1 bis 20 Gew.-% Titan und 50 bis 1.000 ppm eines Elements der Gruppe I-A in oxidischer Form enthält, wobei das Verfahren ausgewählt ist aus einer Gruppe, bestehend aus Copräzipitation, Zersetzung, Imprägnierung und mechanischer Mischung.

9. Verfahren zur Konvertierung von Kohlenmonoxid oder Kohlendioxid zu einem Alkohol in Gegenwart von Wasserstoff und Inertgasen in einem Gasstrom bei einer Temperatur von 200 bis 400°C unter Verwendung des Katalysators nach einem der Ansprüche 1 bis 7.

10. Verfahren zur Entfernung von Spuren von Sauerstoff, Wasserstoff, Chlor und Kohlenmonoxid aus einem Gasstrom unter Verwendung des Katalysators nach einem der Ansprüche 1 bis 7.

11. Hydrierverfahren unter Verwendung des Katalysators nach einem der Ansprüche 1 bis 7.

12. Verfahren zur Konvertierung von Kohlenmonoxid zu Kohlendioxid mit Wasser unter Verwendung des Katalysators nach einem der Ansprüche 1 bis 7.

**Revendications**

1. Catalyseur de conversion de l'oxyde de carbone, qui, avant une activation par réduction, mesurée en poids par rapport au poids sec après calcination à une température d'au moins 370°C, comprend de 30 à 70 % en poids de CuO, de 20 à 50 % en poids de ZnO, de 5 à 40 % en poids d'oxyde d'aluminium, de 0,1 à 20 % en poids d'un oxyde de titane et de 50 à 1000 ppm d'un élément du groupe I-A sous forme d'oxyde.

2. Catalyseur selon la revendication 1, qui comprend de 30 à 60 % en poids de CuO, de 20 à 50 % en poids de ZnO et de 5 à 20 % en poids d'oxyde d'aluminium.

3. Catalyseur selon la revendication 1 ou 2, dans lequel l'élément du groupe I-A est choisi dans l'ensemble comprenant le potassium et le césium..

4. Catalyseur selon l'une quelconque des revendications 1 à 3, qui contient de 0,2 à 10 % en poids d'un oxyde de titane.

5. Catalyseur selon l'une quelconque des revendications 1 à 4, dans lequel, avant la calcination, le titane se présente sous l'une des formes oxyde, hydroxyde, chlorure, sulfate, isopropylate ou propylate.

6. Catalyseur selon l'une quelconque des revendications 1 à 5, dans lequel, avant la calcination, l'élément du groupe I-A se présente sous une forme choisie dans l'ensemble comprenant les hydroxydes, bicarbonates, carbonates, chlorures, bromures, acétates, citrates, oxalates, sulfates et nitrates.

7. Catalyseur selon l'une quelconque des revendications 1 à 6, se présentant sous forme de sphères, de pastilles, de comprimés, d'anneaux, d'extrudats solides creux ou façonnés.

8. Procédé de préparation d'un catalyseur de conversion du monoxyde de carbone qui, avant activation par réduction, mesurée en poids par rapport au poids sec, après calcination à une température d'au moins 370 °C, comprend de 30 à 70 % en poids de CuO, de 20 à 50 % en poids de ZnO, de 5 à 40 % d'$Al_2O_3$, de 0,1 à 20 % en poids de titane et de 50 à 1000 ppm d'un élément du groupe I-A sous forme d'oxyde, où le procédé est choisi dans l'ensemble comprenant une coprécipitation, une décomposition, une imprégnation et un mélange mécanique.

9. Procédé de conversion de monoxyde de carbone ou de dioxyde de carbone en un alcool en présence d'hydrogène et de gaz inertes dans un courant gazeux à une température de 200 à 400°C, par utilisation du catalyseur selon l'une quelconque des revendications 1 à 7.

10. Procédé d'élimination des traces d'oxygène, d'hydrogène, de chlorures et de monoxyde de carbone à partir d'un courant gazeux, par utilisation du catalyseur selon l'une quelconque des revendications 1 à 7.

**11.** Procédé d'hydrogénation utilisant le catalyseur selon l'une quelconque des revendications 1 à 7.

**12.** Procédé de conversion de l'oxyde de carbone en dioxyde de carbone avec de l'eau, utilisant le catalyseur selon l'une quelconque des revendications 1 à 7.